# DEMANDE DE BREVET EUROPEEN

(11) **EP 1 719 478 A2**
(43) Date de publication de la demande: **08.11.2006**
(21) Numéro de dépôt: 06076556.7
(22) Date de dépôt: 18.04.2003
(51) Int. Cl.: A61F 2/38

(54) **Prothèse totale de genou**

(30) Priorité: 19.04.2002 FR 0204946
(62) Demande divisionnaire de: 03290973.1
(71) Demandeur: Score PTG, 69003 Lyon (FR)
(72) Inventeur:
(74) Mandataire: Lavialle, Bruno François Stéphane

(57) **Abrégé**

Prothèse de genou comportant une pièce fémorale (1), une pièce tibiale (2) et un insert (3) interposé entre la pièce fémorale et la pièce tibiale, dans laquelle, le plot (15) de pivotement de l'insert (3) et l'alésage de la pièce tibiale pour le recevoir comporte une partie conique (15a) prolongée d'une extrémité cylindrique (15b).

## Description

La présente invention concerne une prothèse totale de genou, c'est-à-dire formée d'une pièce fémorale, une pièce tibiale et d'un insert interposé entre la pièce tibiale et la pièce fémorale.

L'articulation anatomique du genou est un joint mécanique entre l'armature osseuse de la cuisse (fémur) et l'armature osseuse de la jambe (ensemble tibia péroné) d'un membre inférieur. Ce joint mécanique est d'une cinématique complexe puisque interviennent notamment, une rotation autour d'un axe perpendiculaire au plan sagittal du membre, un déplacement de cet axe parallèlement à lui-même, une rotation-torsion autour d'un axe perpendiculaire au premier intervenant au cours de la rotation précédente, un glissement antéro-postérieur des deux pièces osseuses, chacun de ces degrés de liberté étant plus ou moins bridé ou limité par des ligaments et des tendons qui relient les armatures entre elles directement ou par l'intermédiaire des muscles d'actionnement de l'articulation.

A cette fonction de joint mécanique du genou, il s'ajoute une fonction de «poulie de renvoi à gorge » de l'effort développé par le quadriceps et appliqué à l'armature osseuse de la jambe en contournant la face antérieure du genou par l'intermédiaire du tendon rotulien. La gorge de cette «poulie» coopère au guidage et au maintien de la portion du lien entre le quadriceps et la jambe par l'intermédiaire de la rotule.

Dans cette articulation anatomique, les surfaces qui coopèrent sont cartilagineuses avec d'excellents coefficients de frottement. On y trouve également des pièces d'adaptation mobiles et déformables (par exemple les ménisques) qui ont pour fonction de rendre compliants les différents composants de l'articulation dans tous les états géométriques de l'articulation au regard de la transmission des efforts et de la répartition des pressions entre ces composants.

Lorsqu'on met en place une prothèse de genou, l'articulation ne bénéficie plus de tous les freins et attachements qui existaient naturellement entre les composants en mouvement relatifs ni d'aucune des pièces d'adaptation ou de compliance. Il est donc impossible de reconstruire une articulation de ce genou identique à l'articulation naturelle et toute prothèse n'est qu'une imitation de l'articulation anatomique.

Les prothèses modernes sont de plus en plus proches de l'articulation naturelle, non pas en termes de structure, mais en termes de fonctions assurées. C'est ainsi par exemple que le rôle de compliance des ménisques est joué par un insert qui, sans être aujourd'hui déformable, dispose d'une certaine mobilité aux lieu et place des jeux de fonctionnement entre glène et condyle qui, auparavant, étaient nécessaires à la prothèse pour s'accommoder des trajectoires relatives complexes de la cuisse et de la jambe.

La présente invention ne constitue pas une rupture technologique avec l'état de la technique tel qu'il est illustré par exemple par le document US 5 609 639. Elle concerne en revanche une multitude de perfectionnements et/ou d'améliorations de détails qui conduisent à un optimal s'approchant au plus près des fonctionnalités assurées par une articulation naturelle.

On sait que lors de la flexion de la prothèse, il existe un mouvement antéropostérieur des condyles par rapport à l'insert. On a constaté que dans les zones d'amplitude maximale de ce mouvement et sous l'effet d'un seuil de charge relativement élevé, les forces appliquées à l'insert tendaient à l'expulser, le déboîter, de la pièce tibiale. Pour lutter contre ce risque ou ce défaut, l'invention a pour objet une prothèse de genou comportant une pièce fémorale, une pièce tibiale et un insert interposé entre la pièce fémorale et la pièce tibiale, la pièce fémorale comportant elle-même deux parties condyliennes réunies par une gorge intercondylienne, chaque partie condylienne ayant, dans un plan de coupe sagittal, une section limitée par un profil convexe curviligne et un profil concave polygonal, la pièce tibiale comportant un plateau et une quille sensiblement perpendiculaire au plateau et pourvue d'un alésage central ouvert sur le plateau, l'insert étant limité par une surface plane pourvue d'un plot de pivotement sur la pièce tibiale et, à l'opposé, par une surface complexe définissant deux glènes concaves séparées par un massif antéropostérieur convexe en selle de cheval, dans laquelle le plot de pivotement de l'insert et l'alésage de la pièce tibiale pour le recevoir comportent une partie conique prolongée d'une extrémité cylindrique. La coopération de ces deux extrémités cylindriques lorsque l'insert est en appui sur la pièce tibiale interdit que l'insert bascule sous l'effet d'un effort à forte composante parallèle à l'interligne articulaire, ce qui n'est pas le cas lorsque la coopération entre le plot et l'alésage de la pièce tibiale est réalisée par un emboîtement uniquement conique.

Il sera fait référence aux dessins annexés parmi lesquels :

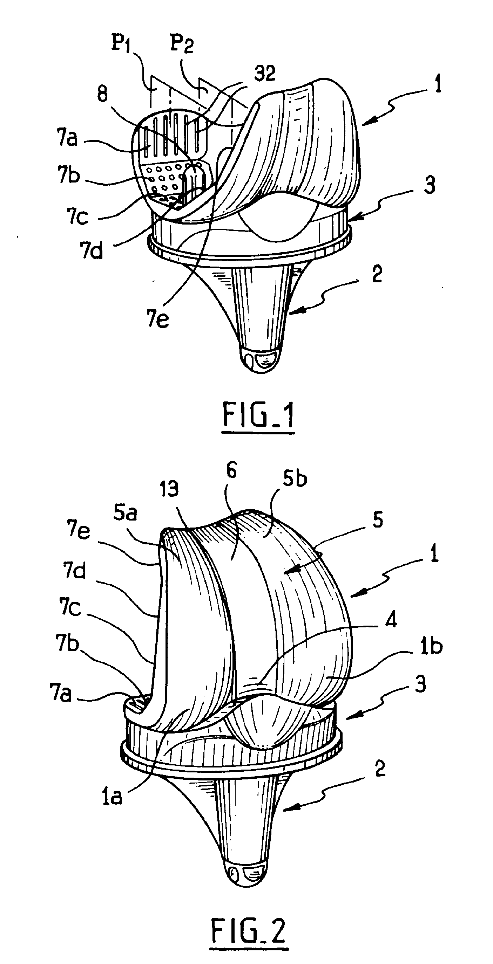

- la figure 1 est une vue de trois quart depuis sa face antérieure d'une prothèse selon l'invention dans une position relative des pièces qui la composent correspondant à l'extension maximale de l'articulation (0° de flexion),
- la figure 2 est une même vue de la prothèse pour une flexion de 90° de l'articulation,

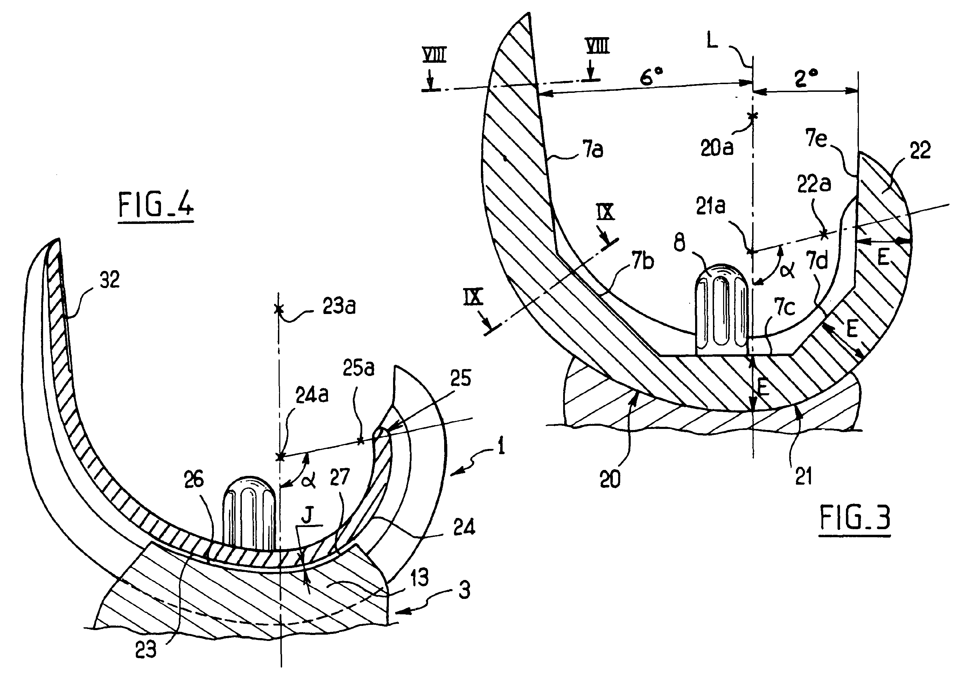

- la figure 3 est une vue extérieure de trois quarts depuis la face antérieure de la prothèse de la pièce tibiale surmontée de l'insert,

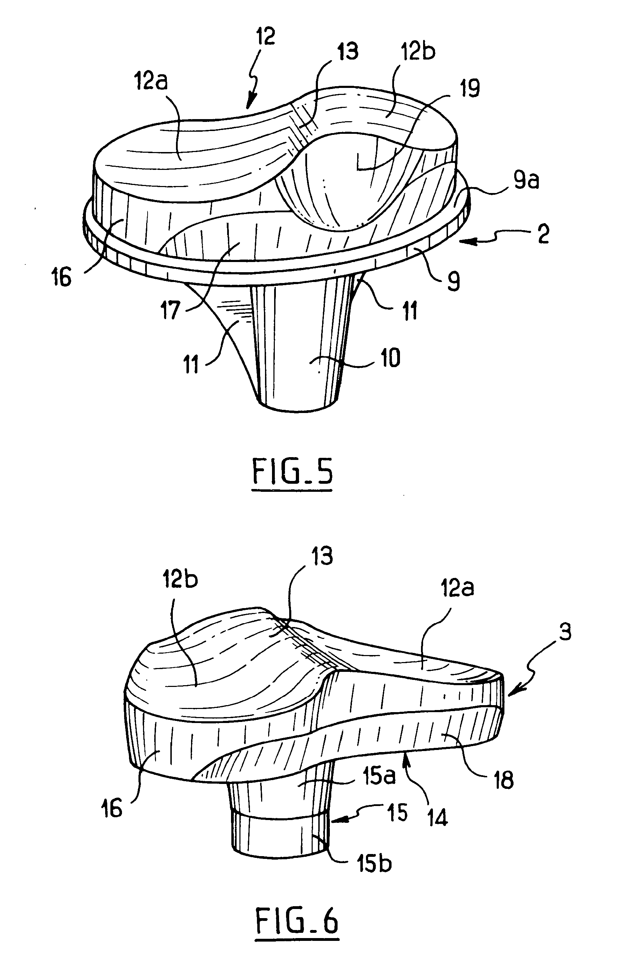

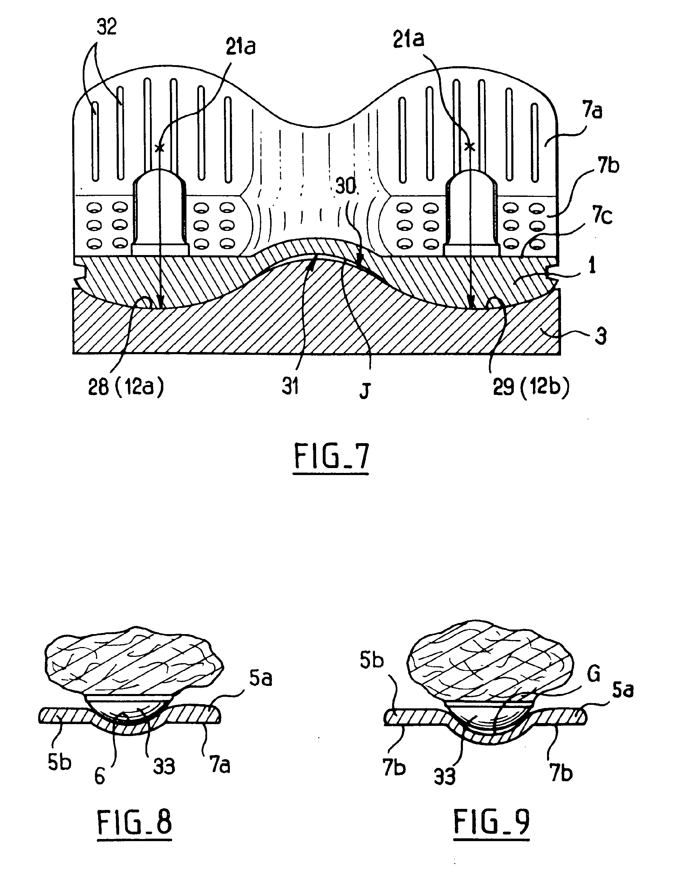

- la figure 4 est une vue de trois quarts de l'insert seul depuis sa face postérieure,

La prothèse représentée aux dessins comporte une pièce fémorale 1, une pièce tibiale 2 et un insert 3. Les pièces fémorale et tibiale sont généralement métalliques tandis que l'insert est en matériau plastique tel que du polyéthylène. La pièce fémorale 1 est globalement divisée en deux parties : une première partie en contact glissant avec l'insert 3 qui comporte un condyle intérieur 1a et un condyle extérieur 1b réunis par une gorge intercondylienne 4. La deuxième partie de la pièce fémorale 1 n'est jamais au contact de l'insert 3 : il s'agit de la partie trochléenne 5 de la pièce fémorale comportant, en prolongement des condyles 1a et 1b des berges intérieure 5a et extérieure 5b séparées par une gorge trochléenne 6. Cette pièce fémorale, en forme de coquille, possède une surface extérieure complexe lisse, globalement convexe et est limitée à l'intérieur par une surface concave formée de cinq faces successives repérées 7a à 7e depuis la partie postérieure jusqu'à la partie antérieure de la pièce fémorale, délimitant un volume prismatique. La face centrale 7c est pourvue de pions d'ancrage 8 de la prothèse dans la tête fémorale réséquée.

La pièce tibiale 2 est de manière classique composée d'un plateau supérieur 9 surfacé (poli-miroir) sur sa face supérieure 9a, et comportant une quille 10 d'implantation dans le tibia avec des ailerons 11, la quille 10 étant creuse pour d'une part être fixée au moyen de cônes morses à une tige implantée dans le tibia et d'autre part, recevoir un plot de pivotement de l'insert 3.

L'insert 3 quant à lui est une pièce en polyéthylène qui présente une surface supérieure 12 complexe divisée en deux glènes 12a, 12b, séparées par un massif antéropostérieur 13. A l'opposé de cette surface supérieure 12, l'insert présente une surface inférieure plane 14 destinée à reposer sur la surface supérieure 9a de la pièce tibiale et dans la partie médiane de laquelle, au droit du massif 13, est implanté un pivot 15 conique dans sa partie 15a proche de la surface 14 et cylindrique à son extrémité 15b. L'alésage prévu pour le recevoir dans la quille 10 de la pièce tibiale 2 est de même forme.

L'insert 3 délimité latéralement par une surface 16 dont le contour est en forme de haricot, comme le contour du plateau tibial et ce de manière connue, cette surface 16 possédant une dépouille antérieure 17 inclinée vers le centre en direction de la surface 14 et une dépouille postérieure 18 également inclinée vers le centre et vers la surface 14. On notera également la présence d'un embrèvement 19 à la jonction du massif antéropostérieur 13 et de la partie antérieure de la surface latérale 16 de l'insert, embrèvement qui sert à dégager un espace de logement du tendon rotulien lorsque l'articulation est en flexion maximale alors qu'il s'est produit un mouvement antéropostérieur de la pièce fémorale par rapport à la pièce tibiale dans cet état.

## Revendications

1. Prothèse de genou comportant une pièce fémorale (1), une pièce tibiale (2) et un insert (3) interposé entre la pièce fémorale et la pièce tibiale, la pièce fémorale comportant deux parties condyliennes (1a, 1b) réunies par une gorge intercondylienne (4) ayant, dans un plan de coupe sagittal, une section limitée par un profil convexe curviligne et un profil concave polygonal, la pièce tibiale comportant un plateau (9) et une quille (10) sensiblement perpendiculaire au plateau et pourvue d'un alésage central ouvert sur le plateau, l'insert étant limité par une surface plane (14) pourvue d'un plot (15) de pivotement sur la pièce tibiale (2) et, à l'opposé, par une surface complexe (12) définissant deux glènes (12a, 12b) concaves, séparées par un massif antéropostérieur (13) convexe en selle de cheval, **caractérisée en ce que** le plot (15) de pivotement de l'insert (3) et l'alésage de la pièce tibiale pour le recevoir comporte une partie conique (15a) prolongée d'une extrémité cylindrique (15b).
